# EUROPEAN PATENT APPLICATION

(11) **EP 1 867 333 A1**
(43) Date of publication of application: **19.12.2007**
(21) Application number: 07117093.0
(22) Date of filing: 08.08.2001
(51) Int. Cl.: A61K 31/5365, A61K 9/20

(54) **Pharmaceutical composition comprising condensed indole compound**

(30) Priority: 08.08.2000 GB 0019524; 02.08.2001 GB 0118919; 03.08.2001 GB 0119022; 07.08.2001 WO PCT/GB01/03544
(62) Divisional of application: 01954214.1
(71) Applicant: SMITHKLINE BEECHAM PLC, Brentford, Middlesex TW8 9GS (GB)
(72) Inventor: Buxton, Philip, Christopher, Harlow, Essex CM19 5AW (GB); Van Schie, Dirk, Marinus, Johannes, Harlow, Essex CM19 5AW (GB); Thomson, Seona, Ware, Hertfordshire SG12 0DP (GB)
(74) Representative: Breen, Anthony Paul

(57) **Abstract**

The invention provides a pharmaceutical composition comprising N-[(1-ⁿbutyl-4-piperidinyl)methyl]-3,4-dihydro-2H-[1,3]oxazino[3,2-a]indole-10-carboxamide (SB 207266) or a pharmaceutically acceptable salt thereof in combination with one or more pharmaceutically acceptable carriers,
wherein at least some of the SB 207266 or the salt thereof is in granulated form, and wherein the SB 207266 or the salt thereof is present in the composition in at least 4 weight % by weight of the composition,
wherein the pharmaceutical composition has been made by a method comprising forming at least some of the SB 207266 or the salt thereof into granules in the presence of a granulating solvent (i.e. using a "wet granulation" process),
and wherein the composition includes an excipient which acts as a granulation aid, and wherein the granulation aid is present inside the granules and is present in at least 15 weight % of the composition.

The granulation aid is preferably microcrystalline cellulose. The granulating solvent can comprise or be water and/or ethanol.

A method of making the pharmaceutical composition is also provided.

## Description

This invention relates to a novel composition, for example a tablet or capsule, comprising SB 207266 or a pharmaceutically acceptable salt thereof.

### Introduction

WO 93/18036 (SmithKline Beecham) discloses a large number of condensed indole compounds as 5-HT4 antagonists including, as Example 3 on pages 17-18, N-[(1-ⁿbutyl-4-piperidinyl)methyl]-3,4-dihydro-2H-[1,3]oxazino[3,2-a]indole-10-carboxamide (SB 207266) and its preferred hydrochloride salt (SB 207266-A). These compounds are disclosed for use in the treatment or prophylaxis of gastrointestinal, cardiovascular and CNS disorders, in particular irritable bowel syndrome. WO 93/18036 also states in the general description on pp.6-7 in general terms that: "Specific cardiac 5-HT₄ receptor antagonists which prevent atrial fibrillation and other atrial arrhythmias associated with 5-HT would also be expected to reduce the occurrence of stroke". See also US 5,852,014, EP 0 884 319 A2, L.M. Gaster et al, J. Med. Chem., 1995, 38, 4760-4763 and Drugs of the Future, 1997, 22(12), 1325-1332 for the compound SB 207266, which is highly selective for the 5HT₄ receptor over other 5HT receptors. The structure of SB 207266 is as follows:

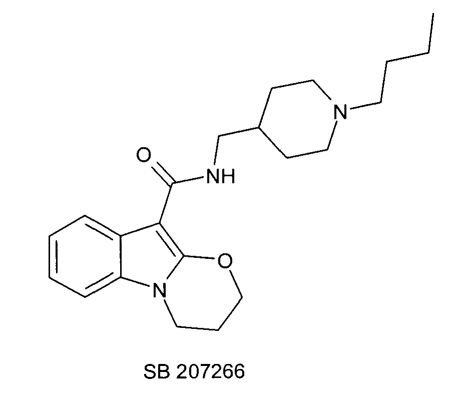

For improved syntheses of SB 207266, see WO 98/07728, WO 98/11067; WO 00/03983; and WO 00/03984.

There are several methods of making the SB 207266 in free base form or as a hydrochloride salt disclosed in the art. Example 3 on page 17-18 of WO 93/18036 discloses the production of SB 207266 in free base form in Methods 1 and 2. Method 2 also discloses conversion to the HCl salt and recrystallisation from ethanol/60-80 petrol to give a white solid. L. Gaster, Drugs of the Future, 1997, 22(12), 1325-1332 discloses a similar method involving HCl salt formation by treatment of SB 207266 free base with anhydrous HCl in ethanol. WO 98/07728 discloses three new methods for making the free base on page 6 line 5 to page 7 line 20. WO 98/07728 also discloses two methods of making the HCl salt (SB 207266 A) - Method A on page 7 line 22 to page 8 line 9, and Method B on page 8 line 10 to page 8 line 19. In page 8 lines 10-19 of WO 98/07728, Method B for making the SB 207266 HCl salt is as follows: "N-[(1-Butyl-4-piperidinyl)methyl]-3,4-dihydro-2H-[1,3]-oxazino[3,2-a]indole-10-carboxamide (SB-207266) (100g, 0.27mol) was dissolved in ethanol (870ml) and the resulting solution filtered to remove particulates. Anhydrous HCl in ethanol (83ml, 3.6M, 0.30mol) was added causing the product to precipitate out of solution. The slurry was heated to redissolve the solid and hexane (550ml) was added. After cooling to room temperature, the mixture was cooled to 0 - 5°C and stirred at that temperature for about two hours. The solid was isolated by filtration and dried *in vacuo* at about 40°C to give the product, N-[(1-butyl-4-piperidinyl)methyl]-3,4-dihydro-2H-[1,3]-oxazino[3,2-a]indole-10-carboxamide hydrochloride, (102.8g) in 94% yield."

### The Invention

It has now been recognised that there are problems with certain processes for making the SB 207266 HCl salt, which processes are similar or identical to the process disclosed as Method B in page 8 lines 10-19 of WO 98/07728 in that the HCl salt is dissolved in ethanol, industrial methylated spirits (IMS, e.g. ethanol containing ca. 1% methanol) or similar and crystallised by addition of a C₅-C₁₀ hydrocarbon (e.g. hexane and/or heptane) and/or a solvent containing a C₅-C₁₀ hydrocarbon (e.g. hexane and/or heptane).

The first aspect of the newly recognised problem is that such processes produce the SB 207266 hydrochloride salt in the form of particles of extremely small particle size. For example, the following table shows the particle size data from batches of SB-207266-A made using a process similar to Method B of page 8 of WO 98/07728 but using heptane instead of hexane in the crystallisation step:

| **Batch** | **DV 90 (µm)** | **DV 50 (µm)** | **DV 10 (µm)** |
|---|---|---|---|
| BDC-H-01C | 12.8 | 5.3 | 1.4 |
| BDC-G-02C | 13.8 | 5.7 | 1.5 |
| BDC-G-03C | 16.4 | 6.8 | 1.8 |
| BDC-G-04C | 14.4 | 5.3 | 1.4 |
| BDC-G-05C | 14.6 | 5.8 | 1.5 |
| Average | 14.4 | 5.8 | 1.5 |

DV 90, DV 50 and DV 10 respectively mean that 90%, 50% and 10% by volume of the material is less than the micron size specified.

The second aspect of the newly recognised problem is the discovery that the SB 207266 HCl salt produced by these processes is very cohesive and has poor flowability / flow characteristics.

The third aspect of the newly recognised problem is that at above certain concentrations in a pharmaceutical formulation, this cohesive drug material causes the composition to be sufficiently poorly-flowing that it cannot easily be tabletted or made into capsules, when the SB-207266 HCl salt is combined with standard methylcellulose, mannitol and Mg stearate excipients. It has been found that a composition for SB 207266, for human oral administration, containing: SB-207266 HCl salt (ca. 5.0 mg), Microcrystalline cellulose (30.0 mg), Mannitol (112.0mg), Mg Stearate (3.0 mg), with total tablet weight = ca. 150 mg, is possible to tablet. However, higher concentrations of the SB-207266 HCl salt are not easily tabletted using this type of formulation.

The fourth aspect of the newly recognised problem is that the small-particle size SB-207266 HCl salt has a low bulk density, densifying on the addition of water. This means that less material can be added to a mixer of fixed volume, leading to a less efficient manufacturing process as large volumes of equipment have to be used for relatively small volumes of drug (smaller throughput in plant).

It has now been discovered that some or all of these problems can be at least partly overcome or mitigated by the forming the SB 207266 HCl salt into granules which have a particle size larger than that of the original SB 207266 HCl salt, e.g. by using a wet granulation process. These granules are found to have better flow characteristics for e.g. tabletting purposes. It has also been found that the incorporation of a filler into the granules, especially an insoluble filler such as CaHPO₄ and/or Ca₃(PO₄)₂, can help to form granules with pharmaceutically advantageous properties, e.g. often minimising dissolution of the very soluble SB 207266 HCl salt in the granulation solvent and so minimising undesirable fusion of granules after removal of the solvent. Some or all of these advantages are also expected to be gained for the free base which is believed also to have usually a small-particle size, e.g. the free base is very slow to filter when crystallised by the addition of hexane to a toluene solution (e.g. as in Method A on page 6 lines 19-23 and Method C on page 7 lines 14-20 of WO 98/07728). Similarly salts other than the HCl salt are thought to benefit too.

Therefore, a first aspect of the invention provides a pharmaceutical composition comprising N-[(1-ⁿbutyl-4-piperidinyl)methyl]-3,4-dihydro-2H-[1,3] oxazino [3,2-a]indole-10-carboxamide (SB 207266) or a pharmaceutically acceptable salt thereof in combination with one or more pharmaceutically acceptable carriers, wherein at least some of the SB 207266 or the salt thereof is in granulated form, and wherein the SB 207266 or the salt thereof is present in the composition in at least 4 weight % by weight of the composition.

Preferably the composition is a tablet, or the invention can be a capsule containing said composition.

Preferably, the granules including the SB 207266 or salt thereof have a particle size defined by a "D50", or mean particle size e.g. by weight (DM50) or by volume (DV50), of ≥ 100 microns (micrometres) e.g. 100 to 1000 microns , more preferably ≥ 200 microns e.g. 200 to 1000 or 200 to 500 microns, still more preferably ≥ 250 microns e.g. 250 to 500 microns. Preferably, 50% by weight or by volume of the granules including the SB 207266 or salt thereof have a particle size of ≥ 100 microns e.g. 100 to 1000 microns, or ≥ 200 microns e.g. 200 to 1000 or 200 to 500 microns, or ≥ 250 microns.

Preferably, the granules including the SB 207266 or salt thereof have a particle size defined by a "D10", e.g. by weight (DM10) or by volume (DV10), of ≥ 10 microns (micrometres) e.g. 10 to 1000 microns, more preferably ≥ 50 microns e.g. 50 to 1000 or 50 to 500 microns, still more preferably ≥ 100 microns e.g. 100 to 500 microns.

Compositions of the invention containing granules with the above-mentioned medium to large particle sizes are generally less cohesive, flow better, and are thus less likely to cause the above-mentioned formulation problems.

Preferably, the particles of the SB 207266 or salt thereof (e.g. before forming into granules and/or after granule formation; e.g. within the granules) have a particle size defined by a "D50", or mean particle size e.g. by weight (DM50) or by volume (DV50), of ≤ 80 microns (micrometres), more preferably ≤ 50 microns, still more preferably ≤ 20 microns, even more preferably ≤ 10 microns, most preferably ≤ 8 microns. Preferably, 50% by weight or by volume of the particles of the SB 207266 or salt thereof (e.g. before forming into granules and/or after granule formation; e.g. within the granules) have a particle size of ≤ 80 microns, e.g. ≤ 50 or ≤ 20 or ≤ 10 or ≤ 8 microns.

Preferably, the particles of the SB 207266 or salt thereof (e.g. before forming into granules and/or after granule formation; e.g. within the granules) have a particle size defined by a "D10", e.g. by weight (DM10) or by volume (DV10), of ≤ 20 microns (micrometres), more preferably ≤ 10 microns, still more preferably ≤ 5 microns, even more preferably ≤ 2.5 microns, most preferably ≤ 2 microns. Preferably, 10% by weight or by volume of the particles of the SB 207266 or salt thereof (e.g. before forming into granules and/or after granule formation; e.g. within the granules) have a particle size of ≤ 20 microns, e.g. ≤ 10 or ≤ 5 or ≤ 2.5 or ≤ 2 microns.

Preferably, the particles of the SB 207266 or salt thereof (e.g. before forming into granules and/or after granule formation; e.g. within the granules) have a particle size defined by a "D90", e.g. DV90 or DM90, of ≤ 100 microns (micrometres), more preferably ≤ 50 microns, still more preferably ≤ 20 microns. Preferably, 90% by weight or by volume of the particles of SB 207266 or salt thereof (e.g. before forming into granules and/or after granule formation; e.g. within the granules) have a particle size of ≤ 100 microns, e.g. ≤ 50 or ≤ 20 microns.

As discussed above, SB 207266 or salts with such small particle sizes are the ones most likely to give the problems above-mentioned, and are most likely to benefit from the present invention.

In general, particle sizes (D50, D10, D90, et al.) can be measured by sieving with one or more sieves (e.g. for granules before further processing into tablets, and/or for measuring the powder inside capsules). For a tablet, particle sizes can be measured directly (e.g. optically e.g. by microscope, or otherwise) in for example a section through the tablet - diameters of specific particles can be measured which enables an estimation of the particle size distribution by volume and thence by weight.

The SB 207266 or the salt thereof is present in the composition in at least 4 weight %, or preferably at least 6 weight % or at least 8 weight %, by weight of the composition. Preferably, the SB 207266 or salt thereof is present in the composition in up to 95 weight %, more preferably up to 70 weight %, most preferably up to 50 weight %. For example, about 10-100 mg (e.g. 10, 20, 25, 40, 50, 75, 80 or 100mg) of SB 207266 or salt thereof (measured either as the free base or as the actual weight including counterions) for every 250mg of weight of composition (e.g. for every 250 mg coated or uncoated tablet weight) is ideal.

Preferably, the N-[(1-ⁿbutyl-4-piperidinyl)methyl]-3,4-dihydro-2H-[1,3]oxazino[3,2-a]indole-10-carboxamide (SB 207266) or the pharmaceutically acceptable salt thereof comprises (e.g. is) the hydrochloride salt of SB 207266 (SB 207266-A).

Preferably, the granules containing the SB 207266 or salt thereof also contain a filler (diluent). Mixing the filler with the SB 207266 or salt thereof before granulation often aids formation of granules. Granulating pure SB 207266 or a salt is difficult.

Preferably, the filler (diluent) is abrasive. This helps to alleviate the cohesiveness of the SB 207266 or salt.

Preferably, the filler (diluent) is insoluble, practically insoluble, very slightly soluble or slightly soluble (more preferably insoluble or practically insoluble, most preferably insoluble) in a/the granulating solvent, e.g. water and/or ethanol. The terms "practically insoluble", "very slightly soluble" and/or "slightly soluble" can be as defined in the British Pharmacopoeia, the European Pharmacopoeia and/or the US Pharmacopoeia. "Practically insoluble" according to the British Pharmacopoeia 1999 (page 11) means that at least 10 litres of the solvent is required to dissolve 1 gram of the filler (e.g. at ambient temperature, e.g. 20 or preferably 25 °C). "Very slightly soluble" according to the British Pharmacopoeia means that at least 1 litre and up to 10 litres of the solvent is required to dissolve 1 gram of the filler (e.g. at 25 °C). "Slightly soluble" according to the British Pharmacopoeia means that at least 100 ml and up to 1 litre of the solvent is required to dissolve 1 gram of the filler (e.g. at 25 °C).

The insoluble, practically insoluble, very slightly soluble or slightly soluble (preferably insoluble) filler, for (wet) granulation, often minimises/reduces undesirable fusion of granules after removal of the granulation solvent and/or improves the quality of the granules. The SB 207266 HCl salt is highly soluble.

Preferably, the filler comprises any pharmaceutically acceptable metal (e.g. calcium or magnesium) salt which is insoluble, practically insoluble, very slightly soluble or slightly soluble (preferably insoluble) in the granulating solvent e.g. water and/or ethanol. The salt can for example be a phosphate, hydrogen phosphate, carbonate or hydrogen carbonate salt. Such insoluble-to-slightly soluble salts include calcium phosphate, dibasic calcium phosphate, calcium carbonate, magnesium carbonate, magnesium phosphate, etc.

Preferably, the filler comprises dibasic calcium phosphate (i.e. dicalcium phosphate, CaHPO₄) more preferably dibasic calcium phosphate hydrate e.g. dihydrate (i.e. CaHPO₄.2H₂O). Anhydrous dibasic calcium phosphate can also be used. CaHPO₄ e.g. hydrated or anhydrous, is abrasive and helps to aleviate the cohesiveness of the SB 207266 or salt; and it is insoluble in water which helps the granulation process as described above. Alternatively or additionally, the filler can comprise calcium phosphate, i.e. tribasic calcium phosphate, Ca₃(PO₄)₂.

The filler is preferably present in up to 95% by weight of the granules and/or up to 70% by weight of the composition. Preferably, the filler is present in ≥ 15 weight % or ≥ 20 weight % or ≥ 30 weight % of the composition. Preferably, the weight ratio of filler to drug in the composition or in the granules is at least 1:3, preferably at least 1:2.5 or at least 1:2 or at least 2:3.

Preferably, the composition includes an excipient which acts as a compression and/or granulation aid, such as microcrystalline cellulose (MCC), preferably present in at least 15 weight %, more preferably 15-30 weight % (e.g. about 20 weight %) of the composition. MCC acts to help plastic deformation when tabletting. The compression and/or granulation aid can be present inside or outside the granules.

Preferably, the composition includes a binder such as hydroxypropylmethylcellulose (HPMC) (e.g. low viscosity HPMC such as Pharmacoat 603). The binder is preferably present in the granules. Other possible binders can include HPC, HEC, HMC, methyl cellulose, ethyl cellulose, etc. The binder can preferably be present in about 2.5 to about 10 weight % (e.g. about 5 weight %) of the composition.

Preferably, the composition includes a disintegrant (e.g. tablet disintegrant) such as sodium starch glycollate. The disintegrant can be preferably present in about 2.5 to about 10 weight % (e.g. about 5 weight %) of the composition.

Preferably, the composition includes a lubricant such as magnesium stearate. The lubricant can be preferably present in about 0.2 to about 2 weight % (e.g. about 1 weight %) of the composition.

A second aspect of the invention provides a method of making a pharmaceutical composition comprising N-[(1-ⁿbutyl-4-piperidinyl)methyl]-3,4-dihydro-2H-[1,3]oxazino[3,2-a]indole-10-carboxamide (SB 207266) or a pharmaceutically acceptable salt thereof in combination with one or more pharmaceutically acceptable carriers, and wherein the SB 207266 or the salt thereof is present in the composition in at least 4 weight % by weight of the composition,
the method comprising forming at least some of the SB 207266 or the salt thereof into granules.

Preferably, the method also comprises mixing some or all of the SB 207266 or salt thereof with a filler and/or a binder before granulation. The filler and/or binder can be as defined above.

Preferably, the granules are formed in the presence of a granulating solvent (i.e. using a "wet granulation" process), e.g. comprising or being water and/or ethanol, preferably water. The solvent can be added after mixing of the SB 207266 or salt with the filler and/or binder. Preferably, just sufficient solvent to enable granulation is used.

Preferably, the solvent is removed after formation of the granules, e.g. by drying.

Preferably, the composition/granules are then optionally mixed with other excipient(s) and compressed into tablets.
SB 207266 or the salt thereof may conveniently be administered by any of the routes conventionally used for drug administration, for instance, parenterally, orally, topically or by inhalation.
Procedures for making the composition and/or tablet and/or capsule may involve mixing, granulating and compressing or dissolving the ingredients as appropriate to the desired preparation.
The excipient(s)/carriers used in the composition should be "pharmaceutically acceptable" in the sense of being compatible with the other ingredients of the formulation and not deleterious to the recipient thereof.
The pharmaceutically acceptable carrier employed may be, for example, either a solid or liquid. Exemplary of solid carriers are lactose, terra alba, sucrose, talc, gelatin, agar, pectin, acacia, magnesium stearate, stearic acid and the like. Exemplary of liquid carriers are syrup, peanut oil, olive oil, water and the like. Similarly, the carrier or diluent may include time delay material well known to the art, such as glyceryl monostearate or glyceryl distearate alone or with a wax.
A wide variety of pharmaceutical forms can be employed. Thus, if a solid carrier is used, the preparation can be tableted, placed in a hard gelatin capsule in powder or pellet form or in the form of a troche or lozenge. The amount of solid carrier will vary widely but preferably will be from about 25mg to about 1g. When a liquid carrier is used, the preparation will be in the form of a syrup, emulsion, soft gelatin capsule, sterile injectable liquid such as an ampoule or nonaqueous liquid suspension.

A particularly preferred oral composition for SB 207266, for human oral administration, is as follows:

| | |
|---|---|
| SB-207266 | 5.0 mg |
| Microcrystalline cellulose | 50.0 mg |
| HPMC | 12.5 mg |
| Sodium Starch glycollate | 12.5 mg |
| Dicalcium phosphate | 167.5 mg |
| Mg stearate | 2.5 mg |
| | |
| Tablet weight | 250 mg |

| | |
|---|---|
| HPMC = hydroxypropylmethylcellulose | |

The dose in the above composition can readily be increased to 20 mg. This composition is the result of a granulation process.

This and other suitable oral compositions for SB 207266 are described in the Examples hereinbelow.
All publications, including but not limited to patents and patent applications, cited in this specification are herein incorporated by reference as if each individual publication were specifically and individually indicated to be incorporated by reference herein as though fully set forth.
The invention will now be described by reference to the following Examples which are merely illustrative and are not to be construed as a limitation of the scope of the present invention.

### EXAMPLES

SB 207266 - N-[(1-ⁿbutyl-4-piperidinyl)methyl]-3,4-dihydro-2H-[1,3]oxazino[3,2-a]indole-10-carboxamide - is made using the synthetic methods described in the introduction, i.e. as described in one or more of WO 98/07728, WO 98/11067; WO 00/03983; and/or WO 00/03984. For the SB 207266 hydrochloride salt, see in particular Method B in page 8 lines 10-19 of WO 98/07728 and minor variations thereof as described above.

### EXAMPLES 1, 2, 3, 3A, 4 and 5 - SB 207266 Pharmaceutical compositions

### Comparative Example 1

An oral composition for SB 207266, for human oral administration, is as follows:

| | |
|---|---|
| SB-207266 | 5.0 mg |
| Microcrystalline cellulose | 30.0 mg |
| Mannitol | 112.0mg |
| Mg Stearate | 3.0 mg |
| | |
| Tablet weight | 150 mg |

This composition is not in accordance with the present invention.

### Example 2

An oral composition for SB 207266, for human oral administration, according to the present invention, is as follows:

| | |
|---|---|
| SB-207266 | 5.0 mg |
| Microcrystalline cellulose | 50.0 mg |
| HPMC (hydroxypropylmethylcellulose) | 12.5 mg |
| Sodium Starch glycollate | 12.5 mg |
| Dicalcium phosphate | 167.5 mg |
| Mg stearate | 2.5 mg |
| | |
| Tablet weight | 250 mg |

The dose in this composition can readily be increased to 20 mg. This composition is the result of a granulation process.

### Example 3

The tablet of Example 2 can be varied by increasing the dose of SB 207266 from 5 mg to up to 20, 60, 75, 80 or 100 mg (measured as the free base), and by decreasing the amount of dicalcium phosphate accordingly while keeping the 250 mg tablet weight constant. The composition can use SB 207266 as the free base or as the hydrochloride salt.

### Example 3A

The compositions of Examples 2 and 3 can use either SB 207266 as the free base or as the hydrochloride salt.

### Example 4 - SB-207266-A Tablets with 10, 25, and 40mg strength (measured as pure free base)

Tablets containing the hydrochloride salt of SB 207266 (SB 207266-A) in amounts of 10, 25 or 40 mg (measured as the free base) were made according to the composition in the table below.

| *Example 4 composition* | | | | |
|---|---|---|---|---|
| *Ingredient* | *Function* | *Quantity (mg*/*tablet)* | | |
| | | 10 mg tablet strength | 25 mg tablet strength | 40 mg tablet strength |
| *Active Ingredient* | | | | |
| SB-207266-A | API | 11.0* | 27.5* | 44.0* |

| *Other Ingredients* | | | | |
|---|---|---|---|---|
| Microcrystalline Cellulose (e.g. Ph. Eur. or NF) | Compression & granulation aid | 50.0 | 50.0 | 50.0 |
| Hydroxypropylmethyl cellulose (e.g. USP) (e.g. Pharmacoat 603) | Binder | 12.5 | 12.5 | 12.5 |
| Sodium starch glycollate (e.g. NF or Ph Eur) | Disintegrant | 12.5 | 12.5 | 12.5 |
| Calcium hydrogen phosphate dihydrate (Dibasic Calcium Phosphate dihydrate) (e.g. Ph. Eur. or USP) | Major diluent | 161.5 | 145.0 | 128.5 |
| Magnesium Stearate (e.g. Ph. Eur. or NF) | Lubricant | 2.5 | 2.5 | 2.5 |
| Purified Water ** (e.g. Ph. Eur. or USP) | Granulating solvent | ** | ** | ** |
| Opadry White YS-1-7003 | Film Coat | 6.25 | 6.25 | 6.25 |
| Purified Water ** | | ** | ** | ** |
| Total Tablet Weight | | 256.25 | 256.25 | 256.25 |

| | | | | |
|---|---|---|---|---|
| * Equivalent to 10, 25, 40mg respectively of pure free base ** Removed during processing | | | | |

The SB-207266-A tablets of Example 4 are packed into high density polyethylene (HDPE) bottles with plastic, child-resistant, induction seal caps.

The formulation used a wet granulation process using an insoluble major excipient, dibasic calcium phosphate dihydrate (or dicalcium phosphate). Dibasic calcium phosphate dihydrate is the major diluent together with microcrystalline cellulose which is added to disperse the granulating solvent and to aid in the overall compressibility. The binding agent added is hydroxypropylmethyl cellulose and the granulation is carried out in a conventional mixer granulator. The granule mix is dried, screened and mixed with sodium starch glycollate as a disintegrant and magnesium stearate as a lubricant to form the compression mix. Tablets are produced on a suitable rotary tablet press, and can be either oval or round in shape.

### Example 4 - Detailed Manufacturing Process, In-process Controls, and Assembly Process

SB-207266-A, microcrystalline cellulose, dibasic calcium phosphate dihydrate, and hydroxypropylmethyl cellulose are blended together. Purified water is added to the blended powders while mixing in a high shear mixer-granulator. The granules are dried in a fluid bed drier and are then transferred to a mixer, where they are blended with sodium starch glycollate and magnesium stearate. The lubricated mix is compressed into tablet cores using a rotary tablet press. The tablet cores are film coated using an aqueous dispersion of Opadry White YS-1-7003.

### Procedure:

1.0 Granulation.
   1.1 Blend the SB-207266, microcrystalline cellulose, hydroxypropylmethyl cellulose and dibasic calcium phosphate dihydrate in a suitable high shear mixer-granulator.
   1.2 Add the purified water to effect the granulation.
   1.3 Dry the granules in a fluid bed drier.
   1.4 Pass the dried granules through a stainless steel screen using a suitable mill.
   1.5 Determine the yield of the granules.
2.0 Manufacture of Compression Mix.
   2.1 Blend the required quantities of sodium starch glycollate and magnesium stearate with the dried granules.
   2.2 Determine the yield of compression mix.
3.0 Tablet Compression.
   3.1 Transfer the compression mix to a suitable tablet machine.
   3.2 Compress the tablets.
   3.3 Determine the yield of the compressed tablets.
4.0 Film Coating.
   4.1 Transfer the tablet cores to a suitable coating machine.
   4.2 Rotate the cores and spray on aqueous dispersion of Opadry.
   4.3 Release test samples are taken randomly from the batch and appropriately labelled.
5.0 Bottle filling
   5.1 HDPE bottles are filled to the appropriate fill count, induction sealed and fitted with a child resistant cap using suitably automated equipment.

### Example 5

In a modification of Example 4, formulations containing 20mg, 50mg, 75 mg, 80 mg and 100 mg SB-207266 (as the hydrochloride salt, but the dose given being measured as the free base) can been used to make tablets. These formulations maintain (a) the total coated tablet weight of 256.25 mg, (b) the total pre-coating tablet weight of 250 mg and (c) the other excipient amounts in the Example 4 compositions, but adjust the amount of dibasic calcium phosphate dihydrate used as the amount of SB 207266 varies. These tablets can be round or oval.

## Claims

1. A pharmaceutical composition comprising N-[(1-ⁿbutyl-4-piperidinyl)methyl]-3,4-dihydro-2H-[1,3]oxazino[3,2-a]indole-10-carboxamide (SB 207266) or a pharmaceutically acceptable salt thereof in combination with one or more pharmaceutically acceptable carriers,
wherein at least some of the SB 207266 or the salt thereof is in granulated form, and wherein the SB 207266 or the salt thereof is present in the composition in at least 4 weight % by weight of the composition,
wherein the pharmaceutical composition has been made by a method comprising forming at least some of the SB 207266 or the salt thereof into granules in the presence of a granulating solvent (i.e. using a "wet granulation" process),
and wherein the composition includes an excipient which acts as a granulation aid, and wherein the granulation aid is present inside the granules and is present in at least 15 weight % of the composition.

2. A composition as claimed in claim 1, wherein the granules including the SB 207266 or the salt thereof have a particle size defined by a "D50", by weight (DM50) or by volume (DV50), of ≥ 100 microns (micrometres).

3. A composition as claimed in claim 1, wherein the granules including the SB 207266 or the salt thereof have a particle size defined by a "D50", by weight (DM50) or by volume (DV50), of 100 to 1000 microns.

4. A composition as claimed in any preceding claim, wherein the granules including the SB 207266 or the salt thereof have a particle size defined by a "D10", by weight (DM10) or by volume (DV10), of ≥ 10 microns (micrometres).

5. A composition as claimed in any of claims 1 to 3, wherein the granules including the SB 207266 or the salt thereof have a particle size defined by a "D10", by weight (DM10) or by volume (DV10), of 10 to 1000 microns.

6. A composition as claimed in any of claims 1 to 3, wherein the granules including the SB 207266 or the salt thereof have a particle size defined by a "D10", by weight (DM10) or by volume (DV10), of 50 to 500 microns.

7. A composition as claimed in any preceding claim, wherein the particles of the SB 207266 or the salt thereof have a particle size defined by a "D50", by weight (DM50) or by volume (DV50), of ≤ 80 microns (micrometres).

8. A composition as claimed in any of claims 1 to 6, wherein the particles of the SB 207266 or the salt thereof have a particle size defined by a "D50", by weight (DM50) or by volume (DV50), of ≤ 50 microns (micrometres).

9. A composition as claimed in any preceding claim, wherein the particles of the SB 207266 or the salt thereof have a particle size defined by a "D10", by weight (DM10) or by volume (DV10), of ≤ 10 microns (micrometres).

10. A composition as claimed in any preceding claim, wherein the particles of the SB 207266 or the salt thereof have a particle size defined by a "D90", either as DV90 or as DM90, of ≤ 100 microns (micrometres).

11. A composition as claimed in any preceding claim, wherein the particles of the SB 207266 or the salt thereof within the granules have a particle size defined in any of claims 7 to 10.

12. A composition as claimed in any preceding claim, wherein the SB 207266 or the salt thereof comprises the hydrochloride salt of SB 207266.

13. A composition as claimed in any preceding claim, wherein the SB 207266 or the salt thereof is the hydrochloride salt of SB 207266.

14. A composition as claimed in claim 12 or 13, wherein the hydrochloride salt of SB 207266 is in a form capable of being made by a process in which the hydrochloride salt is dissolved in ethanol or industrial methylated spirits and crystallised by addition of a C₅-C₁₀ hydrocarbon and/or a solvent containing a C₅-C₁₀ hydrocarbon.

15. A composition as claimed in claim 12 or 13, wherein the hydrochloride salt of SB 207266 has been made by a process in which the hydrochloride salt is dissolved in ethanol or industrial methylated spirits and crystallised by addition of a C₅-C₁₀ hydrocarbon and/or a solvent containing a C₅-C₁₀ hydrocarbon.

16. A composition as claimed in claim 14 or 15, wherein the C₅-C₁₀ hydrocarbon and/or the solvent containing the C₅-C₁₀ hydrocarbon is hexane and/or heptane and/or a solvent containing hexane and/or heptane.

17. A composition as claimed in any preceding claim, wherein the SB 207266 or the salt thereof is in granulated form.

18. A composition as claimed in any preceding claim, wherein the SB 207266 or the salt thereof is present in the composition in at least 6 weight % by weight of the composition.

19. A composition as claimed in any preceding claim, wherein the SB 207266 or the salt thereof is present in the composition in at least 8 weight % by weight of the composition.

20. A composition as claimed in any preceding claim, wherein the SB 207266 or the salt thereof is present in the composition in up to 95 weight %.

21. A composition as claimed in claim 20, wherein the SB 207266 or the salt thereof is present in the composition in up to 70 weight %.

22. A composition as claimed in claim 21, wherein the SB 207266 or the salt thereof is present in the composition in up to 50 weight %.

23. A composition as claimed in any preceding claim, wherein the granules containing the SB 207266 or the salt thereof also contain a filler (diluent).

24. A composition as claimed in claim 23, wherein the filler (diluent) is abrasive.

25. A composition as claimed in claim 23 or 24, wherein the filler (diluent) is insoluble, practically insoluble, very slightly soluble or slightly soluble in the granulating solvent, wherein the granulating solvent is water and/or ethanol.

26. A composition as claimed in claim 25, wherein the filler (diluent) is insoluble or practically insoluble in the granulating solvent which is water and/or ethanol.

27. A composition as claimed in any of claims 23 to 26, wherein the filler comprises any pharmaceutically acceptable metal salt which is insoluble, practically insoluble, very slightly soluble or slightly soluble in the granulating solvent, wherein the granulating solvent is water and/or ethanol.

28. A composition as claimed in claim 27, wherein the salt is a phosphate, hydrogen phosphate, carbonate or hydrogen carbonate salt.

29. A composition as claimed in any of claims 23 to 28, wherein the filler is present in up to 95% by weight of the granules.

30. A composition as claimed in any of claims 23 to 29, wherein the filler is present in up to 70% by weight of the composition.

31. A composition as claimed in any of claims 23 to 30, wherein the filler is present in ≥ 15 weight % of the composition.

32. A composition as claimed in any of claims 23 to 30, wherein the filler is present in ≥ 20 weight % of the composition.

33. A composition as claimed in any of claims 23 to 32, wherein the weight ratio of the filler to the drug (the SB 207266 or the salt thereof) in the composition or in the granules is at least 1:3.

34. A composition as claimed in any of claims 23 to 32, wherein the weight ratio of the filler to the drug (the SB 207266 or the salt thereof) in the granules is at least 1:3.

35. A composition as claimed in any of claims 23 to 32, wherein the weight ratio of the filler to the drug (the SB 207266 or the salt thereof) in the granules is at least 1:2.

36. A composition as claimed in any of claims 23 to 32, wherein the weight ratio of the filler to the drug (the SB 207266 or the salt thereof) in the granules is at least 2:3.

37. A composition as claimed in any preceding claim, wherein the granulation aid is an excipient which acts as a compression and granulation aid.

38. A composition as claimed in any preceding claim, wherein the granulation aid is microcrystalline cellulose.

39. A composition as claimed in any preceding claim, wherein the granulation aid is present in 15 to 30 weight % of the composition.

40. A composition as claimed in claim 39, wherein the granulation aid is present in 20 to 30 weight % of the composition.

41. A composition as claimed in claim 40, wherein the granulation aid is present in 20 weight % of the composition.

42. A composition as claimed in any preceding claim including a binder.

43. A composition as claimed in claim 42, wherein the binder is hydroxypropylmethylcellulose (HPMC), HPC, HEC, HMC, methyl cellulose or ethyl cellulose.

44. A composition as claimed in claim 42, wherein the binder is hydroxypropylmethylcellulose (HPMC).

45. A composition as claimed in claim 44, wherein the binder is low viscosity hydroxypropylmethylcellulose (HPMC).

46. A composition as claimed in claim 42, 43, 44 or 45, wherein the binder is present in 2.5 to 10 weight % of the composition.

47. A composition as claimed in claim 46, wherein the binder is present in 2.5 to 5 weight % of the composition.

48. A composition as claimed in any preceding claim including a disintegrant.

49. A composition as claimed in claim 48, wherein the disintegrant is sodium starch glycollate.

50. A composition as claimed in claim 48 or 49, wherein the disintegrant is present in 2.5 to 10 weight % of the composition.

51. A composition as claimed in any preceding claim including a lubricant.

52. A composition as claimed in claim 51, wherein the lubricant is magnesium stearate.

53. A composition as claimed in claim 51 or 52, wherein the lubricant is present in 0.2 to 2 weight % of the composition.

54. A composition as claimed in any preceding claim being a tablet.

55. A composition as claimed in any preceding claim, wherein the granulating solvent comprises or is water and/or ethanol.

56. A composition as claimed in claim 55, wherein the granulating solvent comprises or is water.

57. A capsule containing a composition as claimed in any of claims 1 to 53.

58. A method of making a pharmaceutical composition comprising N-[(1-ⁿbutyl-4-piperidinyl)methyl]-3,4-dihydro-2H-[1,3]oxazino[3,2-a]indole-10-carboxamide (SB 207266) or a pharmaceutically acceptable salt thereof in combination with one or more pharmaceutically acceptable carriers,
the method comprising forming at least some of the SB 207266 or the salt thereof into granules in the presence of a granulating solvent (i.e. using a "wet granulation" process),
wherein the SB 207266 or the salt thereof is present in the composition in at least 4 weight % by weight of the composition,
and wherein the composition includes an excipient which acts as a granulation aid, and wherein the granulation aid is present inside the granules and is present in at least 15 weight % of the composition.

59. A method as claimed in claim 58, wherein the granules containing the SB 207266 or the salt thereof also contain a filler (diluent) which is insoluble, practically insoluble, very slightly soluble or slightly soluble in the granulating solvent.

60. A method as claimed in claim 58 or 59, wherein the granulating solvent comprises or is water and/or ethanol.

61. A method as claimed in claim 60, wherein the granulating solvent comprises or is water.

62. A method as claimed in claim 58, 59, 60 or 61, wherein the granulating solvent is added after mixing the SB 207266 or the salt thereof with a or the filler and/or a binder.

63. A method as claimed in claim 58, 59, 60, 61 or 62, wherein just sufficient solvent to enable granulation is used.

64. A method as claimed in any of claims 58 to 63, wherein the solvent is removed by drying after formation of the granules.

65. A method as claimed in claim 64, wherein the granules are then mixed with other excipient(s) and compressed into tablets.

66. A method as claimed in any of claims 58 to 65, comprising mixing some or all of the SB 207266 or the salt thereof with a filler (diluent) before granulation.

67. A method as claimed in claim 66, wherein the filler (diluent) is as defined in any of claims 24 to 36.

68. A method as claimed in any of claims 58 to 67, comprising mixing some or all of the SB 207266 or the salt thereof with a binder before granulation.

69. A method as claimed in claim 68, wherein the binder is as defined in any of claims 43 to 47.

70. A method as claimed in any of claims 58 to 69, wherein the granulating aid is an excipient which acts as a compression and granulation aid.

71. A method as claimed in claim 70, wherein the granulation aid is microcrystalline cellulose.

72. A method as claimed in claim 70 or 71, wherein the granulation aid is as defined in any of claims 39 to 41.

73. A method as claimed in any of claims 58 to 72, wherein the composition is as defined in any of claims 2 to 22 and/or any of claims 48 to 54.

74. A pharmaceutical composition as claimed in any of claims 1 to 54, made by a method as defined in any of claims 59 to 73.
